# EUROPEAN PATENT SPECIFICATION

(11) **EP 1 637 547 B1**
(45) Date of publication and mention of the grant of the patent: **29.07.2009**
(21) Application number: 04425690.7
(22) Date of filing: 16.09.2004
(51) Int. Cl.: C08F 16/06, C08F 116/06, C08J 3/24, A61L 15/00, A61L 27/52, A61K 47/32

(54) **Process for the preparation of a polymeric hydrogel based on a highly purified polyvinylalcohol and uses thereof**
Verfahren zur Herstellung eines polymeren Hydrogels auf Basis eines hochreinen Polyvinylalkohols und dessen Verwendung
Procédé de préparation d'un hydrogel d'alcool polyvinylique à haute pureté et son utilisation

(43) Date of publication of application: 22.03.2006
(73) Proprietor: Polymekon S.r.l., 20144 Milano (IT)
(72) Inventor: Protopapa, Carmelo, 72100 Brindisi (IT); Balestrieri, Gerardo, 20100 Milano (IT)
(74) Representative: Bottero, Carlo

(56) References cited:
- WO-A1-00/66191
- WO-A1-02/092678
- US-A- 4 005 040
- US-A- 4 781 926
- US-A- 5 106 876
- C.M.HASSAN, N.A.PEPPAS: "Structure and Morphology of Freeze/Thawed PVA Hydrogels" MACROMOLECULES, vol. 33, 2000, pages 2472-2479, XP002315693

## Description

The present invention relates to the preparation of a completely biocompatible and injectable, crosslinked polymeric hydrogel, based on a polyvinyl alcohol.

Said polymer, which is produced in a highly purified form, revealed itself as particularly suitable for the preparation of a filler to be employed in medicine and cosmetics, so as to fill, correct or treat deficits of different kind, origin and size of the soft tissues.

It also revealed itself useful as a carrier of, for example, proteins, cells and substances provided with a pharmacological activity, in order to promote both the reconstitution and the recovery of the tissues.

Moreover, it also proved useful as a cell culture medium.

It is known how, in the medical field, a number of synthetic and/or natural polymeric substances are more and more used.

Said polymeric substances, also known as biomaterials, are compounds designed for interacting with a biological system and performing a certain function without harming it or being harmed.

Biomaterials are used in permanent implants, or prosthesis, as well in devices or compositions which must contact the human body for a limited time.

The usefulness of said biomaterials is evaluated according to their biofunctionality and biocompatibility.

The biofunctionality relates to the properties required by a device for reproducing a determined biological function from the physical and mechanical point of view.

In turn, the biocompatibility relates to the device ability to keep on performing said function throughout the service life of the implant or administration. The biocompatibility is therefore strictly connected with the interactions between the biomaterial and the tissues contacting therewith.

Substantially, the human body is an aqueous environment at 37°C, with an average pH 5.4. The saline constituting the same is an excellent electrolyte which facilitates the electrolytic corrosion and hydrolysis processes. Moreover, the organism has some processes activated by specific catalysts and specialized cells for isolating, attacking and destroying the foreign bodies.

The human body then forms a very adverse environment for the materials that can be introduced. In turn, the implant made of an exogenous material may initiate reactions which can result also in death; therefore, it is important that implant and host interact in the most appropriate way.

From one side, it is therefore important to find and use materials which are, as far as possible, resistant to the degradation processes from the organism. From the other side, it is as much important to really know the effects that the products resulting from such degradation processes cause on the tissues.

At present, in Medicine and Surgery, for the purpose of correcting deficits in the soft tissues and for the aesthetical reconstruction, different kinds of compounds, various in nature and physical shape, are used.

Among them, it is possible to mention, in particular, the collagen and the hyaluronic acid.

The collagen is very easy to handle, it ensures a certain safety degree in the result and further has low costs.

However, the collagen is a natural protein and therefore is of a certain antigenicity, which not recommend its use in at least 10% of the end users.

Further, also the transiency of the corrective effect (about two months) imparted from the collagen is to be considered not satisfactory.

The hyaluronic acid widely exists in our skin and it is fundamental for the health of dermal cells.

Such compound offers a lower antigenicity than the collagen itself.

However, also in this case, the end product is quickly degradated from the organism enzymes (hyaluronidase) with a consequent loss of the corrective effect.

Other substances, which are characterized in a greater permanency, are used for the same purpose but have complications or risks of different kind.

For example, methacrylates such as dextran are biocompatible but give rise to the formation of unaesthetic palpable, and sometimes painful, small balls. The silicone tends to migrate and may cause chronic inflammations of immune type.

The polyacrylamides may be absorbed and deliver dangerous degradation products (free radicals, monomers and so on) .

The recently introduced interpolymer of an amide-imide type, appears like a safe product but is difficult to use. Particularly, it is not suitable for the correction of small deficits but rather for the reconstruction of serious losses of soft tissue.

So far, therefore, in the medical class there is still the need of a product which provides the proper guarantees of safety, effectiveness and an adequate stability of the result.

Therefore, one of the object of the present invention is to provide to the medical sector a biocompatible and injectable product which can be used as a filler, or replacement, for carrying out the correction of deficits of various nature of the soft tissues.

By way of non limiting example of said deficits above-mentioned, there may be mentioned labial hypoplasia, hypotrophies of the zygomatic and chin area, deficits of tissue resulting from facial hemiatrophies and so on.

The aforesaid and still other objects, which will result apparent from the following detailed description, have been attained by the Applicant, which has found a crosslinked polymeric hydrogel, based on a polyvinyl alcohol, which is non-toxic, stable, of a high purity degree and completely biocompatible. Said hydrogel is the object of the present invention and is in the form of a viscoelastic product which can be injected and shaped in situ, so as to take and maintain for an appropriate lapse of time the form imparted thereto by the plastic surgeon.

Said hydrogel may be produced with different degrees of viscosity (from an approximately thick liquid to an elastic solid) according to the foreseen use and the application place.

Although it is biodegradable, said hydrogel has, however, a high degree of resistance to the enzymatic attack from the organism.

Moreover, said hydrogel is completely lacking in toxicity, as its catabolites do not cause acute or chronic intoxication states, nor cause adverse reactions of a mutagen, carcinogen or immune type.

The base product used for the production of the polymeric crosslinked hydrogel of the present invention is a polyvinyl alcoholic polymer (polyvinylalcohol or PVA, as indicated in short in the following) with a high degree of purity, up to 99% and above.

PVA is a polymeric compound comprised of a distribution of polyvinylalcoholic chains with a different length, or degree of polymerization.

PVA is lacking in toxicity, when used in a highly purified form.

As such, it is employed in medicine, for example as a plasma constituent, in medical devices and so on.

A process for carrying out a PVA-based crosslinked polymeric hydrogel, as described and claimed in the appended claims, is an object of the present invention.

The crosslinked polymeric hydrogel obtained by the process of the invention proved to be completely non-toxic, due to the high degree of purity thereof.

The PVA-based branched polymeric hydrogel, obtained by the process of the present invention, has shown the following unexpected, advantageous features:
- it is completely biodegradable and non-toxic: in fact, it delivers compounds which are easily metabolized from the organism, without displaying adverse reactions;
- it has displayed such a consistency to produce a sufficient volume for carrying out the correction of deficits in soft tissues of different kind;
- it has proved to be adjustable so as to reach the same consistency and elasticity of the tissue to be replaced;
- it has on average a sufficient viscosity for exiting from a syringe and being administrated through needles of different gauges, also the thinnest, still maintaining the desired corrective ability;
- it has such a cross-linking degree to adequately withstand, for a sufficiently long time, the degradation processes initiated from the host tissue.

Said PVA-based crosslinked polymeric hydrogel has such biocompatibility features to correspond to the expected standards from the regulations concerning the controls on the products from the Official Board (ISO 10993, Chapt. V). The corrective effect is reversible, but prolonged, because of the considerable resistance of the hydrogel cross-linking to the enzymatic-type degradation produced by the organism.

Said hydrogel does not contain proteins of animal origin, so it does not stimulate by no means the immune system. Consequently, it is not required to carry out preventive allergologic tests (while it is required when one whishes to employ, for instance, the collagen).

The crosslinked polymeric hydrogel of the present invention has also proved to be stable at room temperature.

As for the length of the corrective effect, in those patients subjected to a treatment with said hydrogel, substantial volumetric decreases of the implant have not been observed, neither after six months and more from the operation.

The aesthetical results have been judged excellent.

In no case the onset of granulomas, allergic responses or intolerance phenomena has been noted.

The PVA-based crosslinked polymeric hydrogel of the present invention is therefore proposed as a completely synthetic and biodegradable, long-lasting reversible filler, absolutely lacking in toxicity and readily injectable.

Its versatility features, combined with those just pointed out, then make it advantageously utilizable as an alternative to the products commonly used at present for the same purpose.

Therefore, it can be appropriately employed in different sectors of the Medicine and Surgery for the purpose of filling the soft tissues, in which deficits both congenital and acquired exist.

Further, it can be suitably employed, in a proper formulation, also as a substances carrier (proteins, cells, drugs and so on) where one wishes to obtain a reparation or a replacement of the tissue.

The use of the PVA-based branched polymeric hydrogel of the present invention as a reversible filler or actives carrier is an object of the present invention, as described and claimed in the appended claims.

The preparation process of the PVA-based crosslinked polymeric hydrogel, comprising inter-chain cross bonds between the PVA polymeric chains, includes:
a) preparing a mixture including an aqueous PVA solution;
b) adding hydrogen peroxide thereto, to obtain an environment rich of ionic charges;
c) subjecting the mixture resulting from passage b) to cryoscopic cross-linking, characterized in that : 10 volumes hydrogen peroxide, in a quantity between 0.1% to 2% wt., based on PVA, or 130 volumes hydrogen peroxide, in a quantity between 0.1% to 0.2% wt. are added during passage b).

The base PVA is preferably selected from those of high purity, commercially available, having an average molecular weight (corresponding to the degree of polymerization) between 400 to 2,500, according to the type of application to which the end product is intended.

Each degree of polymerization corresponds to a different length of the polyvinyl alcoholic chain and then to a different molecular weight and a different intrinsic viscosity of PVA.

In a particularly preferred embodiment, prior to the use, the commercial PVA with high purity above-mentioned is further purified from possible residual acetal groups by means of a boiling water washing process, 80°C to 120°C, or in a water/alcohol mixture, 50°C to 80°C, and then dried in an oven at a temperature between 30°C to 70°C.

Said preliminary washing step ensures the greatest purity (and therefore a nearly insignificant toxicity) to the PVA feed, by allowing the use of a pure product up to 99% and even more.

Alternatively, it is also possible to prepare the desired PVA by means of basic exhaustive hydrolysis of the corresponding polyvinyl ester, preferably the polyvinyl acetate.

Said basic hydrolysis may be carried out by means of processes generally known in the art.

By mere way of example, in order to produce the desired PVA, two parts of the corresponding polyvinyl acetate are dissolved in three parts of methyl alcohol (possibly in the presence of proper quantities of other solvents, such as dimethylformamide or phenol) in a reactor equipped with stirrer and reflux. The hydrolysis of ester groups is performed by adding in the reaction mixture, under stirring and at a solvent boiling temperature, the required quantity of aqueous or alcoholic NaOH or KOH with respect to the existing quantity of polyvinyl acetate. When the hydrolysis proceeds, methyl acetate and PVA are formed. The residual methyl acetate and the solvent are removed from the reaction environment by evaporation. PVA remains, which is dried by obtaining a powder thereof.

Finally, as above described, PVA is washed with water or water/alcohol to give the desired product, which is pure up to 99% and even more.

Preferably, the PVA concentration in the aqueous mixture mentioned at the point a) is between 2% to 30% based on the total weight of the mixture; more preferably, 2% to 12; still more preferably 3% to 8%.

In a preferred embodiment, the PVA mixture above-mentioned at the point a) also includes a quantity of inorganic salts capable of facilitating the formation of inter-chain cross bonds (of a substantially electrostatic type) among the -OH groups of the PVA polymeric chains.

Said salts are preferably selected among A1, Ti, Zr organic complex salts with proper chelating agents, such as for instance glutaraldehyde.

Preferably, the quantity of said salts is between 0.1% to 7% wt, based on PVA; more preferably, 0.5% to 5%.

In another preferred embodiment, the PVA mixture above-mentioned in the point a) also includes a quantity of organic dicarboxylic acid salts, which give rise to the formation of stable inter-chain chemical bonds bridged between the -OH groups of the PVA polymeric chains.

Said salts are preferably selected, for example, among oxalic acid, glutamic acid, glutaric acid, phthalic acid, terephthalic acid metal salts.

The quantity of said salts is varying according to the number of inter-chain stable bonds that one wishes to obtain.

In an embodiment of the invention, the quantity of said dicarboxylic acid salts is between 0.1% to 40% wt based on the PVA.

To a high number of said inter-chain stable bonds, a greater consistency of the end hydrogel and a greater degradation resistance will correspond.

The PVA mixture of the aforesaid point a) may also include a quantity of additional substances, such as physiologically compatible solvents, plasticizers, cross-linkers, excipients.

Among them, there can be mentioned, by way of example, polyethylenglycol, glycerin, inorganic salts such as aluminium sulfate.

The PVA mixture of the aforesaid point a) is made by adding in the aqueous solvent, under stirring, the components at a temperature between 20°C to 100°C, until a complete dissolution.

In the obtained mixture, it is possible to add a proper quantity of hydrogen peroxide.

Said treatment with hydrogen peroxide is carried out by adding 10 volumes hydrogen peroxide in a quantity between 0.1% to 2% wt based on PVA; preferably, 0.2% to 0.5%, or

130 volumes hydrogen peroxide is added in a quantity between 0.1% to 0.2% wt, based on PVA.

The mixture (of the aforesaid point b)) obtained following to the above treatment with hydrogen peroxide is, in turn, subjected to a low temperature cryoscopic cross-linking (or gelation) treatment.

Said cryoscopic cross-linking is carried out by subjecting the mixture of the aforesaid point b) to a refrigeration treatment which is known as freeze-thaw. Said technique substantially consists in the subsequent application of strong cooling cycles and relative heating.

Through the freeze-thaw it has been possible to enhance and increase the formation of dipole-dipole bonds between the -OHs of the PVA polymeric chains.

These per se weak bonds have then been able to bring the PVA chains near with each other, so as to establish in the following more intimate bonds.

In this way, it has been possible to obtain a qualitatively more consistent and stable, general bond strength between the chains.

Thanks to the aforesaid approaching of the PVA polymeric chains, also the formation process of inter-chain cross bonds, caused by the organic complex salts and the dicarboxilyc acid salts above-mentioned, has been greatly facilitated and implemented.

Consequently, the cryoscopic cross-linking obtained by freeze-thaw has allowed to obtain a polymeric hydrogel with a particularly high degree of cross-linking and therefore of resistance.

Preferably, the cryoscopic cross-linking is performed by subjecting the mixture of the above-mentioned point b) to freeze-thaw at temperatures between -4°C to -50°C; preferably, -10°C to -50°C.

The length of said freeze-thaw treatment is between 8 hrs. to 20 hrs.; preferably 10 hrs. to 15 hrs.

When in the PVA mixture of the above-mentioned point b) oxygen is insufflated before carrying out the cryoscopic treatment, the pH of said mixture is adjusted to a value which is slightly lower than 7, for example by means of a phosphate buffer. This allows the formation of small quantities of peroxides within the mixture.

If a greater concentration of peroxides is desired, within said mixture organic acids are further added, such as, by example, lactic acid, oxalic acid, glycolic acid in such quantities not to lower below 5 the pH of the mixture itself.

The treatment with oxygen or hydrogen peroxide allows to obtain an environment particularly rich of ionic charges, which enhance the hooking possibility of the PVA dipoles.

In one of the preferred embodiment, the process of the present invention allows to produce a PVA-based cross-linked polymeric hydrogel having high characteristics on mechanical resistance and elasticity. Such characteristics promote the filling of animal and human tissues, as well the possibility of being able to use this gel as a carrier of pharmacologically active substances, in order to promote, for instance, the reconstitution of tissues.

In fact, according to the need, in the hydrogel of the present invention there can be further incorporated, during the preparation thereof:
- amino acids with an isoelectric point preferably below 6;
- vitamins, such as ascorbic acid;
- acids of natural origin, such as hyaluronic acid, as a carrier;
- proteins;
- cells;
- disinfectants, such as methylene blue, in urology, as a disinfectant of the urinary tract;
- drugs.

Preferably, said additional components are added to the PVA mixture of the above-mentioned point a).

The hydrogels obtained by the application of the freeze-thaw technique to PVA mixture including an amount of organic complex salts proved to be particularly preferred.

The hydrogels obtained by the application of the freeze-thaw technique to PVA mixture including an amount of organic complex salts and an amount of dicarboxylic acid salts proved to be equally preferred.

Besides, these hydrogels further are particularly resistant to the degradation from the organism, thus allowing the preparation of fillers with an above average life.

Rheometric measures on the cross-linked polymeric hydrogels obtained by the process of the present invention have been performed using a controlled stress rheometer ("Rheostress 1"; Haake) by making use of a parallel-plates geometry (Φ = 35 mm) and maintaining a gap of about 1.5 mm.

The optimal rheometric value is resulted between 600 to 1,200 pascals.

All the hydrogels have shown an elastic component greater than the viscous one in the analysed range of frequencies (ω̅).

It has further been observed that the course of the bond strengths, also understood as consistency index, tends to increase with the temperature due to the sterilization.

In fact, the structuring degree, or network force, decreases during the time in the non-sterilized hydrogels, while the contrary occurs in the hydrogels subjected to sterilization.

This represents a further unexpected advantage of the hydrogels made by means of the process of the present invention.

In a preferred embodiment, the hydrogels of the present invention may be prepared through the following procedure of a general validity.
- The PVA is carefully washed in water and alcohol at 60°C in order to purify it from possible residual impurities.
- Next, it is dissolved in bi-distilled water at 90°C under stirring, in the desired proportions, that is:

| | |
|---|---|
| PVA | 2 to 8 weight parts; |
| Bi-distilled water | 98 to 92 weight parts. |

- After complete homogenization, an organic complex salt is added, under stirring, which is selected from A1, Ti or Zr chelates with glutaraldehyde, in the ratio between 0,5% to 5% wt, based on PVA.
- The stirring is continued until the mixture reached the room temperature, then 130 volumes hydrogen peroxide is added, about 0.1% wt based on PVA.
- The resulting mixture is then subjected to the cryoscopic cross-linking, performed by freeze-thaw at temperatures between -4°C to -50°C for 10/12 hrs.
- The formed hydrogel is washed with water, or a water/alcohol mixture, at room temperature.
- Once washed, it is dried in a oven at 37°C.

In another preferred embodiment, the inter-chain cross bonds between the PVA polymeric chains are blocked by a further addition, to the initial aqueous PVA-mixture, of dicarboxylic acids selected from oxalic acid, glutamic acid, glutaric acid, phtalic acid, terephtalic acid, in a quantity between 0.1% to 40% wt based on the PVA.

### EXAMPLE (not in accordance with the invention)

The previously washed PVA is mixed, under stirring, in the shown order, with the following components:

| | |
|---|---|
| bi-distilled water | 79.2 wt % parts; |
| PVA | 20 wt % parts; |
| Polyglycol 6000 | 0.4 wt % parts; |
| Glycerin | 0.1 wt % parts; |
| 10% wt Al₂SO₄.8HO | 0.3 wt % parts. |

The stirring is continued until complete homogenization.

Then, O₂ is bubbled in the mixture for about 3 minutes at room temperature. Finally, the resulting mixture is subjected to freeze-thaw at temperatures between -10°C to -50°C for 15 hrs.

The formed hydrogel is washed with water at room temperature.

Once washed, it is dried in oven at 37°C.

## Claims

1. A process for the preparation of an injectable, PVA-based, crosslinked polymeric hydrogel, including inter-chain cross bonds between the PVA polymeric chains, wherein said process includes:
a) preparing a mixture including an aqueous PVA solution;
b) adding hydrogen peroxide thereto, to obtain an environment rich of ionic charges;
c) subjecting the mixture resulting from passage b) to cryoscopic cross-linking, **characterized in that:**
10 volumes hydrogen peroxide, in a quantity between 0.1% to 2% wt., based on PVA, or 130 volumes hydrogen peroxide, in a quantity between 0.1% to 0.2% wt. are added during passage b).

2. The process according to claim 1, wherein the PVA has an average molecular weight between 400 to 2500.

3. The process according to claim 1, wherein the PVA, prior to the preparation of the mixture of said point a) is further purified.

4. The process according to claim 1, wherein the PVA concentration in the mixture of said point a) is between 2% to 30% wt., based on the total weight of the mixture.

5. The process according to claim 1, wherein the mixture of said point a) further includes a quantity of inorganic or organic salts; said salts are preferably selected from organic complex salts of Al, Ti, Zr with chelating agents, such as glutaraldehyde.

6. The process according to claim 5, wherein said salts are present in a quantity between 0.1% to 7% wt., based on the PVA.

7. The process according to claim 1, wherein the mixture of said point a) further includes a quantity of salts of dicarboxylic organic acids; said salts are preferably selected from the oxalic acid, glutamic acid, glutaric acid, phthalic acid, terephthalic acid metal salts.

8. The process according to one or more of claims 1-7, wherein the mixture of said point a) is prepared by adding in the aqueous solvent, under stirring, the components at a temperature between 20°C to 100°C, until complete dissolution.

9. The process according to claim 1, wherein to the mixture of said point a) 10 volumes hydrogen peroxide, in a quantity between 0.2% to 0.5% wt., based on PVA, is added.

10. The process according to claim 1, wherein said cryoscopic cross-linking is carried out by subjecting the mixture of said point b) to a freeze-thaw treatment, preferably, at temperatures between -4°C to -50°C, for a time between 8 hrs. to 20 hrs.

11. The process according to claim 1, wherein within said hydrogel are further incorporated:
amino acids with an isoelectric point lower than 6; vitamins, such as ascorbic acid;
acids of natural origin, such as hyaluronic acid;
proteins;
cells;
disinfectants, such as methylene blue;
drugs.

12. The injectable PVA-based, crosslinked polymeric hydrogel obtainable by the process of anyone of claims 1-11, **characterized by** a rheometric value between 600 to 1,200 pascals, said value being obtained by rheometric measures performed on said hydrogel using a controlled stress rheometer by making use of a parallel-plates geometry (Φ = 35 mm) and maintaining a gap of about 1.5 mm.

13. Use of the hydrogel according to claim 12, for the preparation of a filler product for the treatment of deficits or pathologies of soft tissues of the human or animal body.

14. The use according to claim 13, wherein said product is a long-lasting reversible filler.

## Patentansprüche

1. Verfahren zur Herstellung eines injizierbaren vernetzten polymeren Hydrogels auf Basis von Polyvinylalkohol (PVA), umfassend Interketten-Vernetzungen zwischen den Polymerketten des PVAs, worin das benannte Verfahren die folgenden Schritte umfasst:
a) Herstellung eines Gemisches umfassend eine wässrige Lösung von PVA;
b) Zusatz von Wasserstoffperoxyd zum Gemisch, um eine Umgebung zu erhalten, die reich an ionischen Ladungen ist;
c) kryoskopische Vernetzung des aus Schritt b) erhaltenen Gemisches, **dadurch gekennzeichnet, daß:**
10 Volumen Wasserstoffperoxyd, in einer Menge von 0,1 bis 2 Gew.-%, in Bezug auf PVA, oder 130 Volumen Wasserstoffperoxyd, in einer Menge von 0,1 bis 0,2 Gew.-% während des Schrittes b) zugesetzt werden.

2. Verfahren nach Anspruch 1, worin das PVA ein durchschnittliches Molekulargewicht von 400 bis 2..500 besitzt..

3. Verfahren nach Anspruch 1, worin das PVA vor der Herstellung des Gemisches des benannten Schrittes a) weiter gereinigt wird.

4. Verfahren nach Anspruch 1, worin die Konzentration an PVA des Gemisches des benannten Schrittes a) von 2 bis 30 Gew.-% liegt, in Bezug auf das Gesamtgewicht des Gemisches.

5. Verfahren nach Anspruch 1, worin das Gemisches des benannten Schrittes a) eine Menge von anorganischen oder organischen Salzen weiter umfasst; die benannten Salze werden bevorzugt aus organischen Komplexsalze von Al, Ti, Zr mit Chelatbildnern, wie Glutaraldehyd ausgewählt.

6. Verfahren nach Anspruch 5, worin die benannten Salze in einer Menge von 0,1 bis 7 Gew.-% in Bezug auf das PVA vorhanden sind.

7. Verfahren nach Anspruch 1, worin das Gemisch des benannten Schrittes a) eine Menge von Salzen von organischen Dicarbonsäuren weiter umfasst; die benannten Salze werden bevorzugt aus Metallsalzen von Oxalsäure, Glutaminsäure, Glutarsäure, Phthalsäure, Terephthalsäure ausgewählt.

8. Verfahren nach einem oder mehreren der Ansprüche 1-7, worin das Gemisch des benannten Schrittes a) hergestellt wird, in dem die Bestandteile unter Rühren ins wässrige Lösungsmittel bei einer Temperatur von 20 bis 100°C bis zur vollständigen Auflösung zugesetzt werden..

9. Verfahren nach Anspruch 1, worin 10 Volumen Wasserstoffperoxyd, in einer Menge von 0,2 bis 0,5 Gew.-%, in Bezug auf PVA, zum Gemisch des benannten Schrittes a) zugesetzt werden..

10. Verfahren nach Anspruch 1, worin die benannte kryoskopische Vernetzung durchgeführt wird, indem das Gemisch des benannten Schrittes b) einer Gefrier-Auftau-Behandlung bevorzugt bei Temperaturen von -4 bis -50°C für eine Zeit von 8 bis 20 Stunden unterworfen wird.

11. Verfahren nach Anspruch 1, worin das benannte Hydrogel die folgenden Bestandteile weiter umfasst:
Aminosäuren mit einem isoelektrischen Punkt unter 6;
Vitamine, wie Ascorbinsäure;
Säuren natürlichen Ursprungs, wie Hyaluronsäure;
Proteine;
Zellen;
Desinfektionsmittel, wie Methylenblau;
Arzneimittel.

12. Injizierbares vernetztes polymeres Hydrogel auf Basis von Polyvinylalkohol (PVA), erhältlich durch das Verfahren nach irgendeinem der Ansprüche 1-11, **gekennzeichnet durch** einen rheometrischen Wert von 600 bis 1.200 Pascal, wobei dieser Wert **durch** rheometrische Messungen auf dem benannten Hydrogel unter Verwendung eines Rheometers mit gesteuerter Beanspruchung mit Parallelplatten-Geometrie (Φ = 35 mm) und unter Aufrechterhaltung einer Spalt von ca. 1,5 mm erhalten wird.

13. Verwendung des Hydrogels nach Anspruch 12 zur Herstellung eines Filler-Produktes für die Behandlung von Mangeln oder Krankheiten der Weichgewebe im Menschen- oder Tierorganismus.

14. Verwendung nach Anspruch 13, worin das benannte Produkt ein langdauernder reversibler Filler ist.

## Revendications

1. Procédé de préparation d'un hydrogel polymère réticulé injectable à base d'alcool polyvinylique (PVA), comprenant des liaisons croisées inter-chaîne entre les chaînes polymères du PVA, où ledit procédé comprend les étapes suivantes:
a) préparer un mélange comprenant une solution aqueuse de PVA;
b) ajouter du peroxyde d'hydrogène au mélange pour obtenir un milieu riche en charges ioniques;
c) soumettre le mélange résultant de l'étape b) à réticulation cryoscopique, **caractérisé en ce que:**
pendant l'étape b) on ajoute peroxyde d'hydrogène 10 volumes, dans une quantité de 0,1% à 2% en poids, sur la base du PVA, ou peroxyde d'hydrogène 130 volumes, dans une quantité de 0,1% à 2% en poids.

2. Procédé selon la revendication 1, où le PVA a un poids moléculaire moyen de 400 à 2.500.

3. Procédé selon la revendication 1, où le PVA est en outre purifié avant la préparation du mélange de ladite étape a).

4. Procédé selon la revendication 1, où la concentration de PVA dans le mélange de ladite étape a) est de 2% à 30% en poids, sur la base du poids total du mélange.

5. Procédé selon la revendication 1, où le mélange de ladite étape a) comprend en outre une certaine quantité de sels inorganiques ou organiques; lesdits sels sont choisis de préférence entre les sels complexes organiques de Al, Ti, Zr avec des chélateurs, tels que glutaraldéhyde.

6. Procédé selon la revendication 5, où lesdits sels sont présents dans une quantité de 0,1% à 7% en poids, sur la base du PVA.

7. Procédé selon la revendication 1, où le mélange de ladite étape a) comprend en outre une certaine quantité de sels d'acides organiques bicarboxyliques; lesdits sels sont choisis de préférence entre les sels métalliques d'acide oxalique, acide glutamique, acide glutarique, acide phtalique, acide téréphtalique.

8. Procédé selon une ou plusieurs des revendications 1-7, où le mélange de ladite étape a) est préparé par addition des composants dans le solvant aqueux sous agitation, à une température de 20°C à 100°C jusqu'à dissolution complète.

9. Procédé selon la revendication 1, où au mélange de ladite étape a) on ajoute peroxyde d'hydrogène 10 volumes, dans une quantité de 0,2% à 0,5% en poids, sur la base du PVA.

10. Procédé selon la revendication 1, où ladite réticulation cryoscopique est réalisée en soumettant le mélange de ladite étape b) à un traitement de congélation-décongélation, préférablement à des températures de -4°C à -50°C, pour une période de 8 heures à 20 heures.

11. Procédé selon la revendication 1, où ledit hydrogel inclut en outre:
des aminoacides ayant un point isoélectrique inférieur à 6;
des vitamines, telles que acide ascorbique;
des acides d'origine naturelle, telles que acide hyaluronique;
des protéines;
des cellules;
des désinfectants, tels que bleu de méthylène;
des médicaments.

12. Hydrogel polymère réticulé injectable à base d'alcool polyvinylique (PVA), pouvant être obtenu par le procédé selon une quelconque des revendications 1-11, **caractérisé par** une valeur rhéométrique de 600 à 1.200 Pascal, ladite valeur étant obtenue par mesurages rhéométriques réalisés sur ledit hydrogel au moyen d'un rhéomètre à imposée en utilisant une géométrie à plaques parallèles (Φ = 35 mm) et maintenant un jeu d'environ 1,5 mm.

13. Utilisation de l'hydrogel selon la revendication 12 pour la préparation d'un produit combleur pour le traitement de déficits ou pathologies des tissus mous de l'organisme humain ou animal.

14. Utilisation selon la revendication 13, où ledit produit est un combleur réversible de longue durée.
